# EUROPÄISCHE PATENTSCHRIFT

(11) **EP 0 433 306 B1**
(45) Veröffentlichungstag und Bekanntmachung des Hinweises auf die Patenterteilung: **16.03.1994**
(21) Anmeldenummer: 89909387.6
(22) Anmeldetag: 23.08.1989
(51) Int. Cl.: A61B 8/12, A61B 1/00, A61M 25/01

(54) **ULTRASCHALLENDOSKOPEINRICHTUNG**
ULTRASONIC ENDOSCOPE SYSTEM
SYSTEME D'ENDOSCOPE A ULTRA-SONS

(30) Priorität: 01.09.1988 DE 3829603
(43) Veröffentlichungstag der Anmeldung: 26.06.1991
(73) Patentinhaber: TomTec Tomographic Technologies Inc., Northbrook, Illinois 60062 (US)
(72) Erfinder: WOLLSCHLÄGER, Helmut, D-7800 Freiburg (DE); WOLLSCHLÄGER, Susanna, D-7800 Freiburg (DE); ZEIHER, Andreas, D-7800 Freiburg (DE); KLEIN, Hans-Peter, D-8017 Ebersberg (DE)
(74) Vertreter: Rackette, Karl, Dipl.-Phys. Dr.-Ing
(86) Internationale Anmeldenummer: DE8900551
(87) Internationale Veröffentlichungsnummer: WO9002520

(56) Entgegenhaltungen:
- EP-A- 61 332
- EP-A- 234 951
- GB-A- 2 143 920
- US-A- 4 655 257
- US-A- 4 686 963

## Beschreibung

Die Erfindung betrifft eine Ultraschallendoskopeinrichtung zum Abtasten eines zu untersuchenden Organes eines Patienten, insbesondere zur Verwendung bei der transösophagealen Echokardiographie, mit einem über ein Ultraschalldiagnostikgerät elektrisch an ein Bildverarbeitungssystem für die Erzeugung multiplanarer Tomogramme anschließbaren Ultraschallwandler, der beweglich im distalen Endbereich des Endoskopes angeordnet ist.

Eine derartige Einrichtung ist aus Roy W. Martin et al, An Endoscopic Micromanipulator for Multiplanar Transesophageal Imaging, Ultrasound in Med. & Biol. Vol. 12, No. 12, pp. 965 - 975, 1986 bekannt und dient zur dreidimensionalen Rekonstruktion eines Ultraschallherzbildes, indem eine Vielzahl von Schnittbildern mit Hilfe eines Ultraschallwandlers aufgenommen wird, der am vorderen Ende eines Endoskopes angebracht ist und um eine quer zur Längsachse des Endoskopes verlaufende Achse verschwenkbar ist. Auf diese Weise ergeben sich Schnittbilder, deren Ebenen schräg zueinander verlaufen. Dies führt dazu, daß die gewonnenen Bilddaten für manche Anwendungen nicht die erforderliche Qualität haben und das Abtastvolumen klein und ungünstig verteilt ist. Insbesondere sind die Abtastungen so ungenau, daß sie schwer reproduzierbar sind, und die dabei erzeugten Abbildungen sich für Vergleichsstudien nicht eignen.

Aus der US-PS 4 327 738 sind ein endoskopisches Verfahren sowie eine Vorrichtung zur Durchführung einer Ultraschall-B-Bild-Abtastung bekannt. Der Ultraschallwandler ist im vorderen starren Endstück eines ansonsten flexiblen Schlauches angeordnet. Um multiplanare Tomogramme zu erzeugen, sind schwer reproduzierbare Lageveränderungen erforderlich, so daß exakte räumliche Rekonstruktionen der abgetasteten Organe nicht möglich sind.

Aus der EP-A1-0 234 951 ist ein Katheter mit einem Ultraschallwandler bekannt, der es gestattet, Blutgefäße abzutasten, indem der Ultraschallwandler um seine Längsachse verschwenkt und/oder in Längsrichtung verschoben wird. Die Reproduzierbarkeit der dabei erhaltenen Tomogramme ist ebenfalls so gering, daß exakte räumliche Rekonstruktionen der abgetasteten Organe nicht möglich sind.

Aus der US-A-4 686 963 ist ein Inspektionsinstrument mit einem gestreckten biegsamen Körper bekannt, der eine Vielzahl von hohlen axial hintereinander angeordneten Gliedern aufweist, die einen Führungskanal für ein fieberoptisches Faserbündel bilden. Durch die Glieder erstreckt sich ein Spanndraht, so daß diese zur Versteifung gegeneinander andrückbar sind.

In der GB-A-2 143 920 ist ein Endoskopschaft beschrieben, der ebenfalls über eine Vielzahl von wirbelartigen Gliedern verfügt, die von einem flexiblen Schlauch umgeben sind.

Ausgehend von diesem Stand der Technik liegt der Erfindung die Aufgabe zugrunde, eine Ultraschallendoskopeinrichtung der eingangs genannten Art zu schaffen, die es gestattet, mit einer hohen Präzision ein großes Volumen im Bereich des zu untersuchenden Organes multiplanar so genau abzutasten, daß die erhaltenen Schnittbilder eine exakte räumliche Rekonstruktion des abgetasteten Organes gestatten.

Diese Aufgabe wird bei einer Endoskopeinrichtung der eingangs genannten Art dadurch gelöst, daß der distale Endbereich des Endoskopes als flexibler Schlauch mit einer den Schlauch umgebenden und als Wasservorlaufstrecke dienenden Ballonhülle ausgebildet ist, die eine Vielzahl von axial hintereinander angeordneten starren Führungsgliedern umgeben und zusammenhalten, welche in Schallaustrittsrichtung einseitig offene Hohlräume mit Seitenwänden aufweisen, durch die ein in axialer Richtung durchgehender Führungskanal für einen den Ultraschallwandler tragenden verfahrbaren Gleitschlitten gebildet ist, der über Führungselemente in den einseitig offenen Hohlräumen geführt ist, daß sich durch die Führungsglieder ein Spanndraht erstreckt, durch den die Führungsglieder mit ihren Stirnflächen gegeneinander andrückbar sind, und daß das proximale Ende des Tubus des Endoskopes mit einer Manipulationseinrichtung verbunden ist, durch die ein dem Spanndraht zugeordneter Bowdenzug sowie ein dem Gleitschlitten zugeordneter Bowdenzug betätigbar sind, und daß die Manipulationseinrichtung einen Wasseranschluß aufweist, durch den Wasser über einen Tubus zum distalen Endbereich förderbar ist.

Dadurch, daß das distale Ende der Ultraschallendoskopeinrichtung als flexibler Schlauch mit einer Vielzahl von starren Führungsgliedern ausgebildet ist, ist es möglich, die Ultraschallendoskopeinrichtung gefahrlos durch gekrümmt verlaufende Körperhöhlen, insbesondere in den Ösophagus eines Patienten vorzuschieben. Wenn die einzelnen starren Führungsglieder mit Hilfe des Spanndrahtes gegeneinander gezogen werden, bilden sie eine starre Führungsschiene für den Gleitschlitten des Ultraschallwandlers, der auf diese Weise über einen längeren Weg präzise verfahren werden kann und es gestattet, eine Vielzahl von parallel zueinander liegender Schichtbilddarstellungen oder Schnittbilddarstellungen zu erhalten. Mit Hilfe des an den Ultraschallwandler anschließbaren Bildverarbeitungssystems ist es möglich, die einzelnen Schnittbilder zu einem räumlichen Gesamtbild zusammenzufassen, das so genau ist, daß interindividuelle Vergleichsuntersuchungen möglich sind. Außerdem ist es infolge der parallelen Ausrichtung der einzelnen Schallebenen senkrecht zur Längsachse der Ultraschallendoskopeinrichtung möglich, die Bewegungen des Herzens und ihre durch pathologische Bedingungen hervorgerufenen Störungen zu erfassen. Dabei ist es möglich, die Aufnahme durch ein EKG zu triggern und nach jeder Herzaktion den Gleitschlitten um ein 256stel des gesamten Verfahrweges zu bewegen, so daß z.B. bei einer Bildmatrixgröße von 256 x 256 Bildpunkten nach 256 Herzaktionen ein Bildwürfel zur Verfügung steht. Ein solcher Datensatz kann im Speicher des Bildverarbeitungssystems gespeichert werden und anschließend in beliebigen Blickrichtungen ohne Auflösungsverlust betrachtet, vermessen und quantifiziert werden. Es ist auch möglich, EKG-getriggert auf jeder Schnittebene einen gesamten Herzzyklus aufzunehmen, so daß am Ende des Abtastweges eine Vielzahl von Bildwürfeln entsprechend der Zahl der Abtastungen in jeder Schnittebene zur Verfügung stehen, die die Beurteilung der Dynamik frei wählbarer Blickrichtungen ermöglichen.

Zur reproduzierbaren Beurteilung der Schnittebenen und zur dreidimensionalen Rekonstruktion der Herzstrukturen aus den gewonnenen Informationen ist es zweckmäßig, die exakte Lage der Ultraschallendoskopeinrichtung und damit der Schallebenen im Körper zu kennen. Wenn der Gleitschlitten mit einer Induktionsschleife versehen wird und außerhalb des Körpers des Patienten zwei rechtwinklig zueinander verlaufende ebene Spulen zur Erzeugung definierter Magnetfelder aufgestellt werden, ist es möglich, die genaue räumliche Lage der Abtastebenen, bezogen auf ein äußeres festes Koordinaten-System, festzustellen. Die Echoinformationen der parallelen zweidimensionalen Schnittebenen können dann entsprechend der Lage der Ultraschallendoskopeinrichtung in absolute Daten des festen dreidimensionalen Koordinatensystems umgerechnet werden.

Durch die Anwendung eines derartigen Verfahrens wird es erstmals möglich, reproduzierbare topographische Informationen über das Herz und seine Bewegungen mittels echokardiographischen Methoden zu erhalten. Durch die Detektion der Schallebeneneinrichtung von außen und Umrechnung der gewonnenen Echoinformation in absolute räumliche Koordinaten sind diese Informationen unabhängig von der Lage des Herzens im Brustkorb und damit geeignet, einerseits zuverlässige Verlaufsuntersuchungen durchzuführen und die gewonnenen Daten für interindividuelle Vergleichsuntersuchungen heranzuziehen. Bewegungen der Speiseröhre im Bezug zum Herzen stören nicht mehr, wenn die jeweilige Lage der Ultraschallendoskopeinrichtung für jede Abtastung bekannt ist und somit eine Lageberücksichtigung bei der Bildauswertung möglich ist. Insgesamt gesehen ergibt sich dank der erfindungsgemäßen Ultraschallendoskopeinrichtung die Möglichkeit einer räumlichen Rekonstruktion des Ultraschallbildes eines Herzens unabhängig von der Lage des Herzens im Brustkorb und unabhängig von der Lage der Speiseröhre zum Herzen. Derartige Untersuchungsdaten sind bisher nur mit erheblich aufwendigeren Verfahren, wie beispielsweise der Angiographie, der Computertomographie und der Kernspintomographie möglich gewesen, wobei diese Verfahren jedoch neben dem Nachteil der höheren Investitionskosten auch den Nachteil der Röntgenstrahlbelastung haben.

Zweckmäßige Ausgestaltungen und Weiterbildungen der Erfindung ergeben sich aus den Unteransprüchen sowie der nachfolgenden Beschreibung eines Ausführungsbeispiels der Erfindung anhand der Zeichnung. Es zeigen:
- Fig. 1: das distale Ende einer erfindungsgemäßen Ultraschallendoskopeinrichtung im gekrümmten Zustand,
- Fig. 2: das distale Ende im versteiften und gestreckten Zustand in einer Schnittansicht,
- Fig. 3: den Gleitschlitten für den Ultraschallwandler in einer vergrößerten Draufsicht,
- Fig. 4: den Gleitschlitten in einer Seitenansicht,
- Fig. 5: einen Querschnitt durch das distale Ende der Ultraschallendoskopeinrichtung in einer etwa fünffach vergrößerten Darstellung,
- Fig. 6: eine perspektivische Ansicht eines in Ultraschallausbreitungsrichtung offenen Führungsgliedes für das distale Ende der Endoskopeinrichtung,
- Fig. 7: einen gegenüber den Fig. 1 und 2 vergrößerten Schnitt durch den zwischen dem distalen Endbereich und dem Tubus der Ultraschallendoskopeinrichtung liegenden Abschnitt und
- Fig. 8: eine Manipulationseinrichtung zur Betätigung der Streck- und Verriegelungseinrichtung für den distalen Endbereich sowie zum Verfahren des Ultraschallwandlers entlang der Längsachse des gestreckten distalen Endbereichs während einer Bildabtastung.

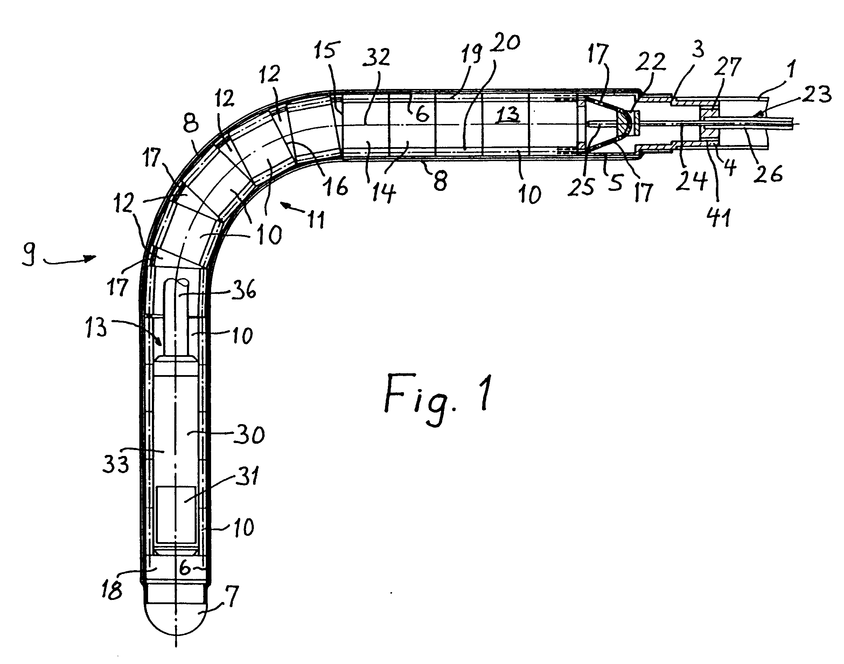

In Fig. 1 erkennt man etwa in natürlicher Größe das einsteckseitige Ende und den distalen Endbereich 9 eines Ösophaguskops oder Ösophagussonde für die Durchführung einer transösophagealen Echokardiographie. Die Ösophagussonde stellt eine Ultraschallendoskopieeinrichtung dar und ist an ein in der Zeichnung nicht dargestelltes Ultraschallbildverarbeitungssystem angeschlossen, um eine Gewinnung räumlicher Informationen über ein untersuchtes Organ, insbesondere das Herz eines Patienten zu gewinnen.

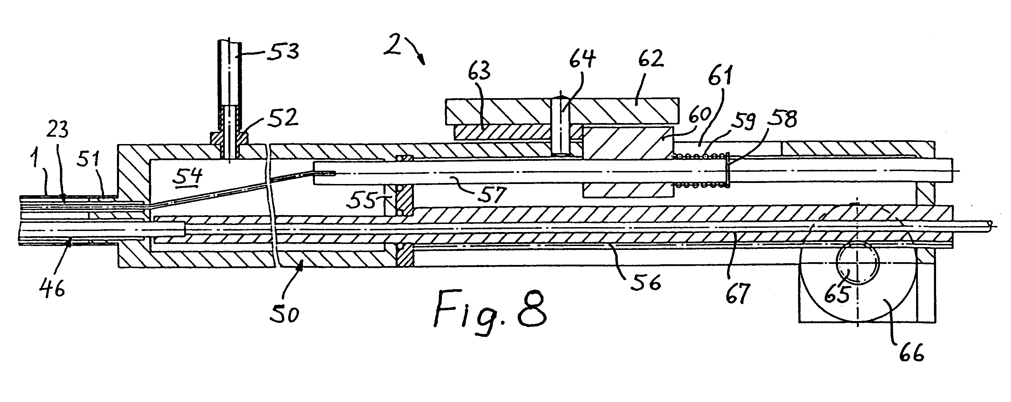

Die Ösophagussonde für die transösophageale Echokardiographie verfügt über einen flexiblen Tubus 1, dessen dem Einsteckende der Ösophagussonde zuwandtes Ende in Fig. 1 zu erkennen ist, und dessen hinteres Ende mit einer in Fig. 8 dargestellten Manipulationseinrichtung 2 verbunden ist.

Wie man in Fig. 1 erkennt, ist das distale Ende des Tubus 1 mit einer Koppelhülse 3 verbunden, die bei dem in Fig. 1 dargestellten Ausführungsbeispiel im wesentlichen aus drei Abschnitten unterschiedlicher Breite besteht, wobei der zylindrische Abschnitt 4 unmittelbar mit dem Tubus 1 verbunden ist.

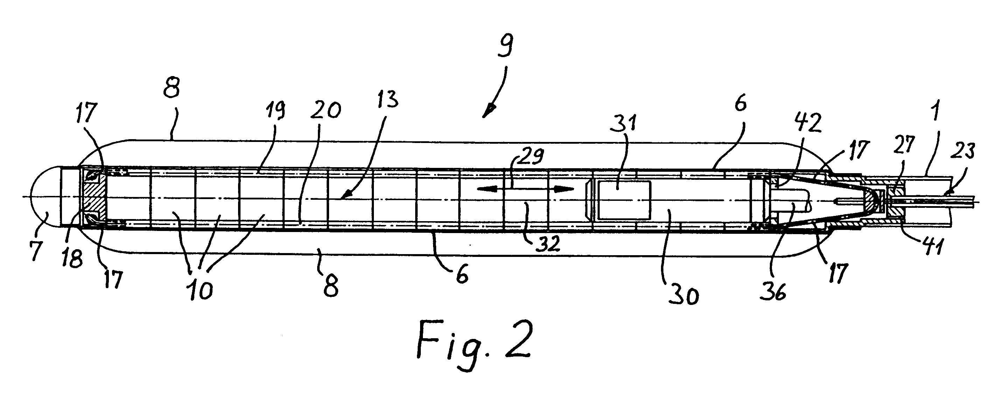

Der dem zylindrischen Abschnitt 4 gegenüberliegende Gehäuseabschnitt 5 ist mit einem flexiblen Schlauch 6, beispielsweise aus Gummi, verbunden, der sich bis zu einem distalen Endstück 7 erstreckt. Der flexible Schlauch 6 ist gegebenenfalls mit in der Zeichnung nicht erkennbaren Perforationen versehen und von einer schlauchförmigen Ballonhülle 8 umgeben, so daß durch Einbringen einer Flüssigkeit, insbesondere Wasser, die Ballonhülle 8 aufgeweitet werden kann, was in Fig. 2 zu erkennen ist.

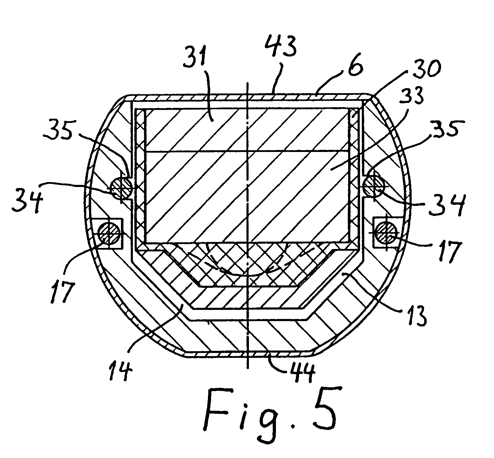

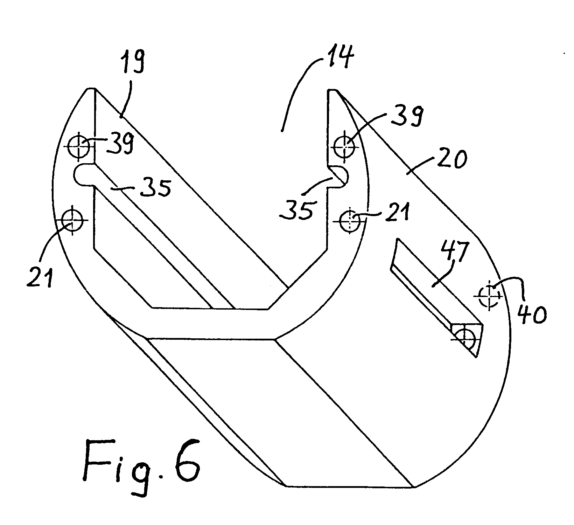

Während in Fig. 1 der distale Endbereich 9 der Ösophagussonde gekrümmt dargestellt ist, ist dieser in Fig. 2 in seiner geraden gestreckten Stellung mit der aufgeweiteten Ballonhülle 8 dargestellt. Wie man in den Fig. 1 und 2 erkennt, umgibt der flexible Schlauch 6 eine Vielzahl von etwa 12 mm langen starren Führungsgliedern 10, deren Gestalt in den Fig. 5 und 6 deutlicher zu erkennen ist. Die Führungsglieder 10 werden durch den flexiblen Schlauch 6 elastisch zwischen dem distalen Endstück 7 und der Koppelhülse 3 festgehalten. Die Gesamtlänge der hintereinander aufgereihten Führungsglieder 10 entspricht dabei dem freien Abstand zwischen dem distalen Endstück 7 und dem Gehäuseabschnitt 5 der Koppelhülse 3.

Wenn auf den elastischen und flexiblen Schlauch 6 quer zu seiner Längsrichtung eine Kraft ausgeübt wird, läßt sich der distale Endbereich 9 der Ösophagussonde in der in Fig. 1 gezeigten Weise verbiegen, wobei ein Krümmungsradius von etwa 35 mm erreichbar ist. Zum Ausgleich der Krümmung bilden sich beim Biegen des distalen Endbereichs 9 zwischen den Führungsgliedern 10 im Krümmungsbereich 11 keilförmige Freiräume 12, die in Fig. 1 gut zu erkennen sind. Aufgrund der durch den flexiblen Schlauch 6 gebildeten Außenhaut und der Aufteilung in viele verhältnismäßig starre und kurze Führungsglieder ist der distale Endbereich 9 flexibel, so daß sich die Ösophagussonde leicht in den Ösophagus eines Patienten einführen läßt.

Wenn die Führungsglieder 10 in der in Fig. 2 dargestellten Weise aufeinandergereiht und sich gegenseitig berührend angeordnet sind, bilden sie einen geraden und stabilen Führungskanal 13, da die Führungsglieder 10 jeweils gleichartig ausgebildete Hohlräume 14 aufweisen, die in den Fig. 5 und 6 deutlicher als in den Fig. 1 und 2 zu erkennen sind.

Um den distalen Endbereich 9 der Ösophagussonde von dem in Fig. 1 dargestellten gekrümmten Zustand in den in Fig. 2 dargestellten gestreckten und versteiften Zustand zu überführen, wird die durch den flexiblen Schlauch 6 aufgebrachte Kraft mit Hilfe einer Streck- und Verriegelungseinrichtung unterstützt, die es gestattet, die Führungsglieder 10 ausgehend von der in Fig. 1 dargestellten gekrümmten Stellung fest gegeneinander anzudrücken, so daß deren distale Stirnflächen 15 und proximale Stirnflächen 16 fest gegeneinander angedrückt werden. Da die distalen Stirnflächen 15 und die proximalen Stirnflächen 16 immer rechtwinklig zur Längsachse der Führungsglieder 10 verlaufen, gestatten es die Führungsglieder 10, den distalen Endbereich 9 mit dem flexiblen Schlauch 6 so zu versteifen, daß der flexible Schlauch 6 von einer gekrümmten Lage in eine präzise gestreckte Lage überführt wird und die Hohlräume 14 der Führungsglieder 10 einen präzisen und gestreckten Führungskanal 13 bilden.

Die Streck- und Verriegelungseinrichtung enthält als wesentliches Element einen Spanndraht 17, der sich, wie in den Fig. 1 und 2 zu erkennen ist, ausgehend von einer in der Nähe des distalen Endstücks 7 angeordneten Spannscheibe 18 des Endstückes 7 durch in den Seitenwänden 19 parallel zu den Längsachsen der Führungsglieder 10 verlaufende Spanndrahtbohrungen 21 erstreckt, die in Fig. 6 zu erkennen sind. Nach dem Austreten des Spanndrahtes 17 aus dem der Koppelhülse 3 benachbarten Führungsglied 10 gelangt der Spanndraht 17 zu einem Umlenkstück 22 und von dort zu den Spanndrahtbohrungen 21 in den den Seitenwänden 19 gegenüberliegenden Seitenwänden 20 der Führungsglieder 10. Dabei erstreckt sich der Spanndraht 17 bis zur Spannscheibe 18, in der beide Enden des im Umlenkstück 22 verschieblich gehaltenen Spanndrahtes 17 befestigt sind.

Das Umlenkstück 22 gestattet es, auf den Spanndraht 17 und somit auf die durch die Seitenwände 19, 20 verlaufenden Spanndrahtabschnitte einen Zug auszuüben, der sich bis zur Spannscheibe 18 fortpflanzt und dadurch alle Führungsglieder 10 in Richtung auf die Koppelhülse 3 zieht. Auf diese Weise läßt sich durch Rückziehen des Umlenkstückes 22 und Spannen des Spanndrahtes 17 der distale Endbereich 9 aus einer gekrümmten Lage in eine gestreckte Lage überführen und durch Fixieren des Umlenkstücks 22 in der zurückgezogenen Lage verriegeln.

Wie man in den Fig. 1, 2 und 7 erkennt, ist das Umlenkstück 22 zum Ausüben einer Zugkraft auf die beiden Abschnitte des Spanndrahtes 17 mit einem Bowdenzug 23 verbunden, dessen Zugkabel 24 sich durch eine Bohrung im Umlenkstück 22 erstreckt und mit einer Zughülse 25 gesichert ist. Die Außenhülle 26 des Bowdenzuges 23 ist in einer Querwand 27 in der Koppelhülse 3 gelagert, so daß über den Bowdenzug 23 mit Hilfe der in Fig. 8 dargestellten Manipulationseinrichtung 2 eine Zugkraft zum Ausrichten und Fixieren des distalen Endbereichs 9 ausgeübt werden kann.

In dem durch die Führungsglieder 10 gebildeten Führungskanal 13 ist ein in den Fig. 1 bis 4 dargestellter Gleitschlitten 30 verfahrbar. In Fig. 1 ist der Gleitschlitten 30 in der vorgeschobenen und in Fig. 2 in der zurückgezogenen Stellung dargestellt. Der Gleitschlitten 30 ist innerhalb des Führungskanals 13, wenn dieser sich in der gestreckten, in Fig. 2 dargestellten Konfiguration befindet, in Richtung des Doppelpfeils 29 um etwa 130 mm verschiebbar. Der Gleitschlitten 30 trägt einen Ultraschallwandler 31, beispielsweise einen Gruppenstrahler, der es gestattet, eine rechtwinklig zur gestreckten Längsachse 32 des distalen Endbereichs 9 verlaufende Ebene mit Ultraschall abzutasten, wobei für die Abtastebene mit Hilfe des Ultraschallbildverarbeitungssystems, das an die Ösophagussonde angeschlossen ist, ein Ultraschallschnittbild in Gestalt einer tomographischen Schichtbilddarstellung erfassbar ist.

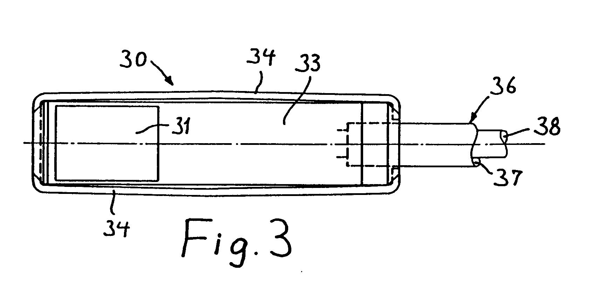

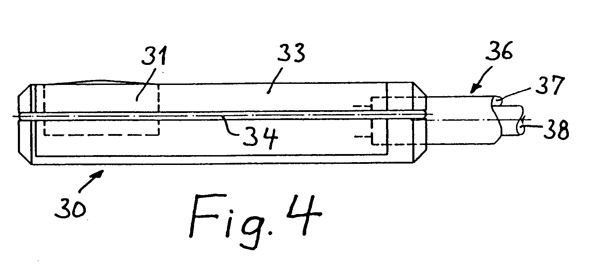

Der Ultraschallwandler 31 ist im Gleitschlitten 30 in einer Vergußmasse 33 eingebettet. Wie man in den Figuren 3 und 4, die den Gleitschlitten 30 gegenüber den Figuren 1 und 2 etwa zweifach vergrößert darstellen, erkennt, ist der Gleitschlitten 30 mit einem Führungsbügel 34 versehen, der es gestattet, den Gleitschlitten 30 in dem einseitig offenen Führungskanal 13 der Führungsglieder 10 präzise in Richtung des Doppelpfeils 29 zu führen und zu verschieben. Dazu sind in den Führungsgliedern 10 in den Figuren 5 und 6 erkennbare Führungsnuten 35 vorgesehen, in die die Schenkel des Führungsbügels 34, der beispielsweise aus einem elastischen Draht besteht, eingreifen.

Der Ultraschallwandler 31 ist über ein vieladriges Kabel 36, das mit dem Gleitschlitten 30 im distalen Endbereich 9 sowie im Tubus 1 in Längsrichtung verschiebbar ist, verbunden. Das vom Gleitschlitten 30 wegweisende Ende des Kabels 36 mündet in die Manipulationseinrichtung 2, die es gestattet, das Kabel 36 vorzuschieben und zurückzuziehen. Außerdem ist das Kabel 36 elektrisch mit dem Ultraschallbildbearbeitungssystem verbunden, um für eine Vielzahl von Positionen des Gleitschlittens 30 und damit für eine Vielzahl von parallel zueinander verlaufenden Ebenen Schnittbilder der Umgebung des distalen Endbereichs 9 der Ösophagussonde aufzunehmen.

Das Kabel 36 weist eine flexible Schub- und Zughülle 37 auf, die ein elektrisches Leitungsbündel 38 umgibt.

Wie man den Figuren 3 bis 6 entnehmen kann, erstrecken sich die Schenkel des Führungsbügels 34, die Führungsnuten 35 und die Spanndrahtbohrungen 21 parallel zur Längsachse 32 des distalen Endbereichs 9 der Ösophagussonde. Um die Führung des Führungsbügels 34 in den Führungsnuten 35 besonders günstig zu gestalten, kann in der in Fig. 3 dargestellten Weise vorgesehen sein, daß der Bügel geringfügig bogenförmig oder leicht konvex gestaltet ist, um einen geringen seitlichen Druck auf die Seitenwände 19 und 20 der Führungsglieder 10 auszuüben. Auf diese Weise wird eine besonders sichere Führung erreicht, wenn der Gleitschlitten 30 zur Erfassung einer Vielzahl von Schnittbildern ausgehend vom vorderen Ende des distalen Endbereichs 9 in Richtung zum proximalen Ende gezogen wird. Alternativ ist es auch möglich, umgekehrt zu verfahren, d.h. beim Abtasten einen Vorschub auszuführen.

Da zum Versteifen des distalen Endbereichs 9 nur ein einziger Spanndraht 17 verwendet wird, dessen beiden Enden an der Spannscheibe 18 befestigt sind, können Ungleichmäßigkeiten besonders gut ausgeglichen werden. Dazu ist es lediglich erforderlich, daß der Spanndraht 17 sich im Umlenkstück 22 geringfügig quer verschiebt. Um das genaue Fluchten der Führungsglieder 10 nach dem Ziehen am Bowdenzug 23 zu unterstützen, sind die distalen Stirnflächen 15 oder die proximalen Stirnflächen 16 mit Erhebungen 39, beispielsweise in Gestalt von Zentrierkegeln versehen, denen in der jeweils benachbarten proximalen Stirnfläche 16 oder benachbarten distalen Stirnfläche 15 Vertiefungen 40 zugeordnet sind, wobei die Lage der Erhebungen 39 und Vertiefungen 40 genau so gewählt ist, daß die Erhebungen 39 eines Führungsgliedes 10 in die Vertiefungen 40 des benachbarten Führungsgliedes 10 eingreifen. In Fig. 6 erkennt man weiterhin eine Ausnehmung 47, die es gestattet, die Spanndrahtbohrungen 21 kurz zu halten und damit leichter herzustellen.

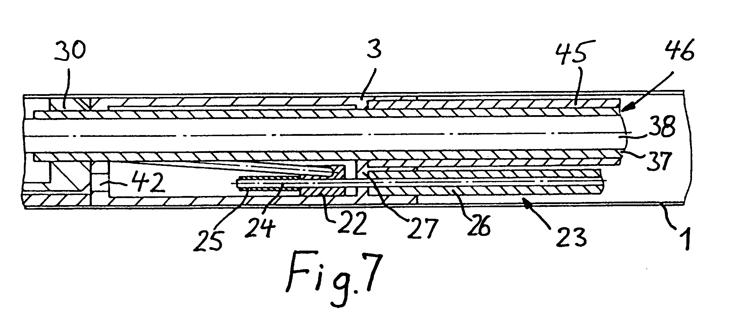

Während Fig. 6 ein einzelnes nach oben offenes Führungsglied 10 ohne den umgebenden flexiblen Schlauch 6 und den eingesetzten Gleitschlitten 30 zeigt, verdeutlicht der in Fig. 5 dargestellte Querschnitt durch den distalen Endbereich 9 einer Ösophagussonde ohne die oben erwähnte Ballonhülle 8 die gegenseitige Anordnung der verschiedenen Bauteile. Der um den Gleitschlitten 30 im Führungskanal 13 verbleibende Hohlraum 14 ist beim Einsatz der Ösophagussonde mit Wasser gefüllt, um eine gute Schallankopplung zu erreichen. Die Wasserzufuhr geschieht über den Tubus 1 sowie in den Figuren 1 und 2 erkennbare Durchgänge 41 in der Querwand 27. Zusätzliche Verbindungskanäle 42 für die Wasserzufuhr sind am distalen Ende der Koppelhülse 3 vorgesehen, was beispielsweise in Fig. 7 dargestellt ist. Wenn abweichend von der in Fig. 5 dargestellten Ausführungsform eine Ballonhülle 8 vorgesehen ist, befinden sich in der Oberseite 43 des Schlauches 6 Perforationen, durch die Wasser in den Innenraum der Ballonhülle 8 gelangen kann.

Wie sich insbesondere aus den Figuren 5 und 6 ergibt, haben die Führungsglieder 10 einen im wesentlichen U-förmigen Querschnitt, wobei die Seitenwände 19 und 20 nach außen gekrümmte schalenförmige abgerundete Flächen bilden. Oben sind die Führungsglieder 10 offen und an der Unterseite 44 sind sie etwas abgeflacht. Die Hohlräume 14 in den Führungsgliedern 10 sind entsprechend der Gestalt des Gleitschlittens 30 ausgebildet und haben vorzugsweise parallel verlaufende Innenseiten, die in Richtung auf die Unterseite 44 rinnenartig zusammenlaufen. Auf diese Weise wird eine besonders kompakte Ausgestaltung erreicht, so daß der Außendurchmesser der Ösophagussonde kleingehalten werden kann.

Wie man in Fig. 7 erkennen kann, bildet das Kabel 36 zusammen mit einem Kabelschlauch 45 einen weiteren Bowdenzug 46 zum Vorschieben und Zurückziehen des auf dem Gleitschlitten 30 angeordneten Ultraschallwandlers 31.

Der Kabelschlauch 45 des Bowdenzugs 46 endet ebenso wie die Außenhülle 26 des Bowdenzugs 23 im Gehäuse 50 der Manipulationseinrichtung 2, die in Fig. 8 in einem Querschnitt etwa in natürlicher Größe dargestellt ist. Das Gehäuse 50 verfügt über einen Anschlußstutzen 51, der mit Bohrungen für die Bowdenzüge 23 und 46 sowie mit in Fig. 8 nicht erkennbaren Bohrungen für die Wasserzufuhr versehen ist und auf dessen Außenmantel der Tubus 1 der Ösophagussonde aufgeschoben ist.

Wie man in Fig. 8 erkennt, verfügt die Manipulationseinrichtung 2 über einen Wasseranschlußstutzen 52, der mit einem Wasserschlauch 53 für die Wasserzufuhr zur Unterstützung der Ultraschallankopplung verbunden ist. Wie man der Zeichnung weiter entnehmen kann, mündet der Wasseranschlußstutzen 52 in den Innenraum 54 der Manipulationseinrichtung 2, die eine Zwischenwand 55 aufweist, durch die sich einerseits eine Zahnstange 56 und andererseits eine Zugstange 57 erstreckt. Die dazu vorgesehenen Öffnungen in der Zwischenwand 55 sind zur Vermeidung des Austritts von Wasser mit O-Ringen 58 und 59 abgedichtet.

Das Zugkabel 24 des Bowdenzugs 23 zum Versteifen und Ausrichten des distalen Endbereichs 9 ist mit dem in Fig. 8 linken Ende der Zugstange 57 verbunden. Die Zugstange 57 ist im Gehäuse 50 längsverschieblich geführt. Wie die Fig. 8 weiter zeigt, ist auf der Zugstange 57 ein Anschlagring 58 befestigt, gegen den eine Druckfeder 59 anliegt, der ein auf der Zugstange 57 verschiebbares Andruckstück 60 zugeordnet ist. Das Andruckstück 60 ragt durch einen Schlitz 61 aus dem Gehäuse 50 nach außen und kann mit Hilfe eines Handrades 62 dem ein Exenter 63 zugeordnet ist, ausgehend von der in Fig. 8 dargestellten linken Endlage nach rechts verschoben werden, wenn das Handrad 62 mit dem Exenter 63 um die am Gehäuse 50 befestigte Achse 64 verdreht wird. Beim Verschieben der Zugstange 57 nach rechts erfolgt eine Kraftübertragung über den Bautenzug 23 auf den Spanndraht 17 und dadurch ein Strecken und Versteifen des distalen Endbereichs 9.

Die im Gehäuse 50 längsverschieblich geführte Zahnstange 56 steht mit einem Ritzel 65 in Eingriff, das mit Hilfe eines Schrittmotors 66 antreibbar ist. Je nach der Drehrichtung des Schrittmotors 66 wird die Zahnstange 56 verschoben und dabei der Bowdenzug 46, der mit dem in Fig. 8 linken Ende der Zahnstange 56 verbunden ist, betätigt. Die flexible Schub- und Zughülle 37 des Kabels 36 ist mit der Zahnstange 56 fest verbunden. Das elektrische Leitungsbündel 38 des Kabels 36 erstreckt sich durch einen Kabelkanal 67 der Zahnstange 56 und verläßt die Manipulationseinrichtung 2 auf der in Fig. 8 rechts gezeigten Seite des Gehäuses 50 und der Zahnstange 56. Der Schrittmotor 66 wird durch den im Bildverarbeitungssystem enthaltenen Rechner gesteuert, wobei die Schrittweite, die Zeitpunkte des Weiterbewegens und die Verzögerungen gegenüber einem aus einem EKG abgeleiteten Triggerimpuls einstellbar sind. Die Schrittweite ist so gewählt, daß die einzelnen Schnittebenen 0,5 mm Abstand voneinander haben.

## Patentansprüche

1. Ultraschallendoskopeinrichtung zum Abtasten eines zu untersuchenden Organs eines Patienten, insbesondere zur Verwendung bei der transösophagealen Echokardiographie, mit einem über ein Ultraschalldiagnostikgerät elektrisch an ein Bildverarbeitungssystem für die Erzeugung multiplanarer Tomogramme anschließbaren Ultraschallwandler, der beweglich im distalen Endbereich des Endoskopes angeordnet ist, **dadurch gekennzeichnet,** daß der distale Endbereich (9) des Endoskopes als flexibler Schlauch (6) mit einer den Schlauch (6) umgebenden und als Wasservorlaufstrecke dienenden Ballonhülle (8) ausgebildet ist, die eine Vielzahl von axial hintereinander angeordneten starren Führungsgliedern (10) umgeben und zusammenhalten, welche in Schallaustrittsrichtung einseitig offene Hohlräume (14) mit Seitenwänden (19, 20) aufweisen, durch die ein in axialer Richtung durchgehender Führungskanal (13) für einen den Ultraschallwandler (31) tragenden verfahrbaren Gleitschlitten (30) gebildet ist, der über Führungselemente (34, 35) in den einseitig offenen Hohlräumen (14) geführt ist, daß sich durch die Führungsglieder (10) ein Spanndraht (17) erstreckt, durch den die Führungsglieder (10) mit ihren Stirnflächen (15, 16) gegeneinander andrückbar sind, und daß das proximale Ende des Tubus (1) des Endoskopes mit einer Manipulationseinrichtung (2) verbunden ist, durch die ein dem Spanndraht (17) zugeordneter Bowdenzug (23) sowie ein dem Gleitschlitten (30) zugeordneter Bowdenzug (46) betätigbar sind, und daß die Manipulationseinrichtung (2) einen Wasseranschluß (52) aufweist, durch den Wasser über einen Tubus (1) zum distalen Endbereich (9) förderbar ist.

2. Ultraschallendoskopeinrichtung nach Anspruch 1 **dadurch gekennzeichnet,** daß die Führungsglieder (10) zwischen einer Spannscheibe (18) und einer Koppelhülse (3) entlang zweier Abschnitte des Spanndrahtes (17) angeordnet sind, der sich durch Bohrungen (21) in zwei einander gegenüberliegenden Seitenwänden (19, 20) der Führungsglieder (10) ersteckt und in seinem mittleren Bereich (22) an einen Bowdenzug (23) angekoppelt ist.

3. Ultraschallendoskopeinrichtung nach Anspruch 1 oder 2, **dadurch gekennzeichnet,** daß die Führungsglieder (10) an ihren Stirnflächen (15, 16) einander zugeordnete Erhebungen (39) und Vertiefungen (40) aufweisen, die im versteiften und gestreckten Zustand des distalen Endbereichs (9) mit denen eines benachbarten Führungsgliedes (10) in Eingriff stehen.

4. Ultraschallendoskopeinrichtung nach einem der vorstehenden Ansprüche, **dadurch gekennzeichnet,** daß die Seitenwände (19, 20) der Führungsglieder (10) auf der zum Führungskanal (13) weisenden Innenseite Führungsnuten (35) aufweisen, durch die der Gleitschlitten (30) des Ultraschallwandlers geführt ist.

5. Ultraschallendoskopeinrichtung nach Anspruch 4, **dadurch gekennzeichnet,** daß der Gleitschlitten 30 einen beidseitig überstehenden Führungsbügel (34) aufweist, der in die beiden seitlichen Führungsnuten (35) eingreift.

6. Ultraschallendoskopeinrichtung nach Anspruch 5, **dadurch gekennzeichnet,** daß der Führungsbügel (34) leicht konvex gebogen und elastisch ist.

7. Ultraschallendoskopeinrichtung nach einem der vorstehenden Ansprüche, **dadurch gekennzeichnet,** daß der flexible Schlauch (6) Perforationen aufweist.

8. Ultraschallendoskopeinrichtung nach Anspruch 1, **dadurch gekennzeichnet,** daß der dem Spanndraht (17) zugeordnete Bowdenzug (23) mit einer axial verschiebbaren Zugstange (57) verbunden ist, die mit Hilfe eines Exenter (63) verschiebbar und arretierbar ist.

9. Ultraschallendoskopeinrichtung nach Anspruch 1 oder 8, **dadurch gekennzeichnet,** daß der dem Gleitschlitten (30) zugeordnete Bowdenzug (46) mit einer durch einen Schrittmotor (66) betätigbaren Zahnstange (56) verbunden ist, durch die der Gleitschlitten (30) und der Ultraschallwandler (31) zu Beginn der Abtastfolge vorschiebbar und nach jeder Abtastung um einen vorherbestimmten Betrag in Richtung auf das proximale Ende des Endoskopes zurückziehbar oder vorschiebbar sind.

## Claims

1. An ultrasound endoscope device for scanning an organ to be investigated of a patient, in particular for use in transoesophagal echocardiography, comprising an ultrasound transducer which can be electrically connected by way of an ultrasonic diagnostic apparatus to an image processing system for producing multi-planar tomograms and which is arranged movably in the distal end region of the endoscope, characterised in that the distal end region (9) of the endoscope is in the form of a flexible hose (6) with a balloon casing (8) which surrounds the hose (6) and which serves as a water feed section and which surrounds and holds together a plurality of rigid guide members (10) which are arranged axially one behind the other and which have cavities (14) that are open at one side in the sound outlet direction, with side walls (19, 20), thereby forming a guide passage (13) which is continuous in the axial direction for a movable slide carriage (30) which carries the ultrasound transducer (31) and which is guided by way of guide elements (34, 35) in the cavities (14) that are open at one side, that extending through the guide members (10) is a tensioning wire (17) by which the guide members (10) can he pressed with their end faces (15, 16) against each other, and that the proximal end of the tube (1) of the endoscope is connected to a manipulation means (2) by which a Bowden cable (23) associated with the tensioning wire (17) and a Bowden cable (46) associated with the slide carriage (30) can he actuated, and that the manipulation means (2) has a water connection (52) through which water can he conveyed by way of a tube (1) to the distal end region (9).

2. An ultrasound endoscope device according to claim 1 characterised in that the guide members (10) are arranged between a tensioning disc (18) and a coupling sleeve (3) along two portions of the tensioning wire (17) which extends through bores (21) in two mutually oppositely disposed side walls (19, 20) of the guide members (10) and is coupled in its middle region (22) to a Bowden cable (23).

3. An ultrasound endoscope device according to claim 1 or claim 2 characterised in that the guide members (10) have at their end faces (15, 16) mutually associated raised portions (39) and recesses (40) which in the stiffened and extended condition of the distal end region (9) engage with those of an adjacent guide member (10).

4. An ultrasound endoscope device according to one of the preceding claims characterised in that the side walls (19, 20) of the guide members (10) have on the inside that faces the guide passage (13) guide grooves (35) by which the slide carriage (30) of the ultrasound transducer is guided.

5. An ultrasound endoscope device according to claim 4 characterised in that the slide carriage (30) has a guide loop (34) which projects at both sides and which engages into the two lateral guide grooves (35).

6. An ultrasound endoscope device according to claim 5 characterised in that the guide loop (34) is slightly convexly curved and elastic.

7. An ultrsound endoscope device according to one of the preceding claims characterised in that the flexible hose (6) has perforations.

8. An ultrasound endoscope device according to claim 1 characterised in that the Bowden cable (23) associated with the tensioning wire (17) is connected to an axially displaceable pull rod (57) which can he displaced and arrested by means of an eccentric (63).

9. An ultrasound endoscope device according to claim 1 or claim 8 characterised in that the Bowden cable (46) associated with the slide carriage (30) is connected to a rack (56) which can be actuated by a stepping motor (66) and by which the slide carriage (30) and the ultrasound transducer (31) can he advanced at the beginning of the scanning sequence and after each scanning operation can be retracted or advanced by a predetermined amount towards the proximal end of the endoscope.

## Revendications

1. Système d'endoscope à ultra-sons pour explorer un organe à examiner sur un patient, en particulier pour être utilisé en échocardiographie transoesophagienne, comportant un convertisseur à ultra-sons pouvant être raccordé électriquement, par l'intermédiaire d'un appareil de diagnostic à ultrasons, à un système de traitement de l'image pour obtenir des tomographies en plusieurs plans, le convertisseur à ultra-sons étant mobile à l'extrémité distale de l'endoscope, caractérisé en ce que la zone d'extrémité distale (9) de l'endoscope est constituée par un tuyau flexible (6) avec une enveloppe de ballon (8) entourant le tuyau et servant de voie d'accès à l'eau, entourant et maintenant ensemble un alignement axial d'éléments de guidage (10) rigides présentant, dans la direction de sortie des ultrasons, des cavités (14) à parois latérales (19, 20) ouvertes d'un côté, formant ensemble un conduit de guidage (13) continu dirigé dans le sens axial, pour le coulisseau (30) mobile portant le convertisseur à ultra-sons (31), qui est guidé par des éléments de guidage (34, 35) à l'intérieur des cavités (14) ouvertes d'un côté, en ce qu'un câble de tension (17) passe à travers les éléments de guidage (10), permettant aux éléments de guidage (10) d'être serrés les uns contre les autres par leurs faces frontales (15, 16), en ce que l'extrémité proximale du tube (1) de l'endoscope est reliée à un manipulateur (2) grâce auquel peuvent être actionnés un câble Bowden (23) associé au câble de tension (17), ainsi qu'un câble Bowden (46) associé au coulisseau (30), et en ce que le manipulateur (2) est équipé d'une alimentation en eau (52) par laquelle de l'eau peut être envoyée par un tube (1) à la zone d'extrémité distale (9).

2. Système d'endoscope à ultra-sons selon la revendication 1, caractérisé en ce que les éléments de guidage (10) sont disposés entre un anneau de tension (18) et un manchon de couplage (3) le long d'une seconde portion du câble de tension (17), qui s'étend par des perçages (21) situés dans deux parois latérales (19, 20) opposées des éléments de guidage (10) et qui est raccordé dans sa portion médiane (22) à un câble Bowden (23).

3. Système d'endoscope à ultra-sons selon l'une des revendications 1 et 2, caractérisé en ce que les éléments de guidage (10) comportent sur leurs faces frontales (15, 16) des bossages (39) et des empreintes (40) correspondant entre eux, qui, dans l'état rigide et rectiligne de la zone d'extrémité distale (9) coïncident avec ceux de l'élément de guidage (10) adjacent.

4. Système d'endoscope à ultra-sons selon l'une quelconque des revendications précédentes, caractérisé en ce que les parois latérales (19, 20) des éléments de guidage (10) comportent, sur leur face intérieure, côté conduit de guidage (13), des rainures de guidage (35) servant à guider le coulisseau (30) du convertisseur à ultra-sons (31).

5. Système d'endoscope à ultra-sons selon la revendication 4, caractérisé en ce que le coulisseau (30) présente un arceau de guidage (34) débordant de chaque côté et qui est en prise avec les deux rainures de guidage (35).

6. Système d'endoscope à ultra-sons selon la revendication 5, caractérisé en ce que l'arceau de guidage (34) est légèrement convexe et élastique.

7. Système d'endoscope à ultra-sons selon l'une quelconque des revendications précédentes, caractérisé en ce que le tuyau flexible (6) présente des perforations.

8. Système d'endoscope à ultra-sons selon la revendication 1, caractérisé en ce que le câble Bowden (23) associé au câble de tension (17) est relié à une barre de traction (57) susceptible de se déplacer dans le sens axial, et pouvant être déplacée et bloquée en position sous l'action d'un excentrique (63).

9. Système d'endoscope à ultra-sons selon l'une ou l'autre des revendications 1 et 8, caractérisé en ce que le câble Bowden (46) associé au coulisseau (30) est relié à une crémaillère (56) actionnée par un moteur pas-à-pas (66) et par laquelle le coulisseau (30) et le convertisseur à ultra-sons (31) peuvent être déplacés pour débuter la séquence exploratoire, puis décalés, après chaque exploration, d'une valeur prédéterminée en avançant ou en reculant par rapport à l'extrémité proximale de l'endoscope.
